# NEUE EUROPÄISCHE PATENTSCHRIFT

(11) **EP 0 289 639 B2**
(45) Veröffentlichungstag und Bekanntmachung des Hinweises auf die Entscheidung über den Einspruch: **11.12.1996**
(45) Hinweis auf die Patenterteilung: 14.08.1991
(21) Anmeldenummer: 87106650.2
(22) Anmeldetag: 07.05.1987
(51) Int. Cl.: A61K 7/06, A61K 31/455

(54) **Verwendung von Trigonellin zum Wiederbeleben und zum Anregen und Verstärken des Haarwuchses**
Use of trigonellin to revive, to stimulate and to enhance hair growth
Utilisation de la trigonelline pour ranimer, stimuler et renforcer la pousse des cheveux

(43) Veröffentlichungstag der Anmeldung: 09.11.1988
(73) Patentinhaber: Mai, Jutta, D-78224 Singen (DE); Mai, Heinz, D-78224 Singen (DE)
(72) Erfinder: Mai, Jutta, D-7700 Singen-Bohlingen (DE)
(74) Vertreter: Weiss, Peter, Dr. rer.nat.

(56) Entgegenhaltungen:
- WO-A-90/12560
- DE-A- 3 118 882
- DE-A- 3 225 056
- DE-A- 3 300 491
- DE-A- 3 603 601
- FR-A- 2 551 972
- THE MERCK INDEX, 7. Auflage, 1960, Merck & Co. Inc. Verlag, US
- CHEMICAL ABSTRACTS, Band 94, Nr.13, 30 März 1981, Columbus, OH (US); F.SZEGO et al., Seite 381, Nr.109080t
- CHEMICAL ABSTRACTS, Band 79, Nr.4, 30 Juli 1973, Columbus, OH (US); E.MARTTINEN et al., Seite 330, Nr.23508s
- CHEMICAL ABSTRACTS, Band 95, Nr.10, 07 September 1981, Columbus, OH (US); SUNSTAR INC., Seite 329, Nr.86147z
- ARCON-Tisane, Anzeige der Firma Arcon GmbH, Böhlingen, im Südkurier vom 24.11.90
- G. Fischer et al. "Heilkräuter und Arzneipflanzen" Haug Verlag, 1984, S. 56-57
- R. Willfort "Gesundheit durch Heilkräuter", R. Trauner Verlag, 1969, S. 270-271

## Beschreibung

Die Erfindung betrifft eine Möglichkeit der Verwendung von Trigonellin.

Trigonellin ist beispielsweise wesentlicher Bestandteil des Samens der Pflanze Bockshornklee (trigonella phoenum graecum). Die aus dem Samen gewonnene Droge wird schon in alter Kräuterliteratur als stärkend, blutbildend und blutreinigend sowie als gutes Magenmittel hervorgehoben. Äußerlich wird sie als Gurgelwasser, bei Halsentzündungen, zum äußerlichen Behandeln von Geschwüren als Kataplasma verwendet. In Form von Waschungen oder Teilbädern wirkt sie gegen Hautjucken und bei Hämorrhoidalbeschwerden findet sie als Klistier Anwendung (siehe G. Fischer/E. Krug, Heilkräuter und Arzneipflanzen, Seite 56-57, Haug-Verlag, 1984).

Aus der DE-A 3 225 056 ist ein oral zu verabreichendes Arzneimittel bekannt, dessen bevorzugte Ausführungsform ein gefriergetrocknetes Produkt des Samens von Bockshornklee zur Bekämpfung toxischer Leberschädigungen und/oder zur Verbesserung der Leberfunktion bzw. der protektiven Wirkung auf die Leber ist. Als zu erzielende Wirkung soll insbesondere eine Beschleunigung des Blutalkoholabbaus eintreten.

Aus dem Lehrbuch "Gesundheit durch Heilkräuter" von Richard Willfort, 1969, Seiten 270/271 wird als Heil- und Wirkstoff des Bockshornklees unter anderem das Alkaloid Trigonellin erwähnt. Als Beispiele konkreter Anwendung wird neben einer großen Zahl äußerer Anwendungsmöglichkeiten, wie z.B. Auflösen von Geschwülsten, Linderung bei Neuralgien, offenen Füßen usw., unter anderem aufgeführt, daß Bockshornkleesamen auch innerlich als kalt angesetzter Tee bei Schleimbildung, Schwächezuständen, Rachities, diversen tuberkulösen Erkrankungen sowie allgemein bei entzündlichen Vorgängen des Rachens und der Mundschleimhaut als Gurgelwasser wirkt. Äußerlich wird der Samenaufguß bei Haarschwund und Erbgrind als Badewasser empfohlen. Gleichfalls soll sich eine Wirkung zeigen, wenn die Kopfhaut mit zerstoßenem Samen und Olivenöl zu einem Brei vermengt eingerieben wird. Dies unterbricht den Haarausfall und läßt neue Haare wieder wachsen, wenn nicht tiefere Ursachen den Haarausfall auslösen.

Ferner wird in der FR-A 2 551 972 der Einsatz von Bockshornkleesamen in einer Mischung von zehn pflanzlichen Stoffen in Form einer Einreibe-Lösung ("Lotion") zur topischen Behandlung des Haarausfalls und zum Wiederwachsenlassen des Haares erwähnt. Zur Anwendung wird die Kopfhaut mit besagter Lösung eingerieben und dieselbe dann mehrere Stunden einwirken gelassen. Nach vierfacher Anwendung soll der Haarausfall vollständig gestoppt sein und die Haare wieder wachsen.

Die Erfinder haben sich zum Ziel gesetzt, eine neue Verwendung von Trigonellin zu finden.

Die Lösung der Aufgabe besteht in der Verwendung von Trigonellin zum Herstellen eines peroral einzunehmenden, kapselierten Mittels zur Wiederbelebung und zum Anregen und Verstärken des Haarwuchses bei Lebewesen.

Das Ausdünnen oder Zurückfliehen des Haarwuchses ist meist auf eine zu starke Straffung des Kopfhaut zurückzuführen, wodurch die Zirkulation des Blutes zu den Haarfolikeln unterbrochen oder unterbunden wird. Die zurückgehende Zufuhr von Nährstoffen und Sauerstoff zu den Haarfolikeln hat einen ungünstigen Einfluß auf die Produktion des Proteins Keratin, welches wesentlich für das Haarwachstum verantwortlich ist. Die Erfinder haben sich bei der erfindungsgemäßen Verwendung des Trigonellin dessen Wirkung auf eine Erweiterung der Blutgefäße zunutze gemacht, wodurch eine bessere Lieferung von Nährstoffen zu den Haarfolikeln möglich wird. Die Haarfolikeln werden wiederbelebt, wobei die Wiederbelebung von einer Hypertrichose, d.h. Verlängerung, Verdickung und erhöhter Pigmentierung des Haares, begleitet ist.

Das Trigonellin kann künstlich hergestellt sein. Bevorzugt wird aber ein Extrakt aus Pflanzen der Unterfamilie Trigonella und hier insbesondere aus Bockshornklee (trigonella phoenum graecum). Dieser Extrakt kann in besonders wirkungsvoller Weise aus dem Samen des Bockshornklees gewonnen werden, indem man den Samen aufquellen läßt und dann mit Wasser extrahiert.

Wird dem Trigonellin-Extrakt Vitamin B6 zugegeben, verstärkt sich die Wirkung des Mittels, da Vitamin B6 ein Hauptnährstoff für das Haar ist und damit zu einem gesunden Haarwachstum beiträgt.

Weiterhin sollte dem Extrakt ein Alkohol und ein Konservierungsmittel zugegeben werden. Hierdurch erhält man ein Breitspektrumtonikum.

Das Trigonellin kann andererseits auch mit einem weiteren wässrigen Bockshornklee-Extrakt gemischt werden, wodurch nicht nur seine Wirkung verbessert, sondern auch sein Wirkungsspektrum verbreitert wird.

Das Mittel wird in Kapseln verpackt und dient zur oralen Einnahme.

Für die kapselierte Form wird in der Regel auf die Anteile Alkohol und Nipastat verzichtet. Stattdessen wird die vitaminisierte Form von Riboflavin, Nicotinamid, Calciumpantothenat und Folsäure angeboten.

## Patentansprüche (Patentansprüche für folgende(n) Vertragsstaat(en): AT, BE, CH, ES, FR, GB, GR, IT, LI, LU, NL, SE)

1. Verwendung von Trigonellin zum Herstellen eines peroral einzunehmenden, kapselierten Mittels zur Wiederbelebung und zum Anregen und Verstärken des Haarwuchses bei Lebewesen.

2. Verwendung von Trigonellin nach Anspruch 1, wobei Trogonellin zusammen mit Vitamin B6 verwendet wird.

3. Verwendung nach Anspruch 1 oder 2, wobei Trigonellin zusammen mit Riboflavin und/oder Nicotinamid und/oder Calciumpantothenat und/oder Folsäure verwendet wird.

4. Verwendung von Trigonellin nach einem der Ansprüche 1 bis 3, wobei das Trigonellin aus dem Samen des Bockshornklees (trigonella phoenum graecum) gewonnen wird.

## Patentansprüche (Patentansprüche für folgende(n) Vertragsstaat(en): DE)

1. Verwendung von Trigonellin zusammen mit Riboflavin und/oder Nicotinamid und/oder Calciumpantothenat und/oder Folsäure zum Herstellen eines peroral einzunehmenden, kapselierten Mittels zur Wiederbelebung und zum Anregen und Verstärken des Haarwuchses bei Lebewesen.

2. Verwendung von Trigonellin nach Anspruch 1, wobei das Trigonellin aus dem Samen des Bockshornklees (trigonella phoenum graecum) gewonnen wird.

3. Verwendung von Trigonellin nach Anspruch 1 oder 2, wobei das Trigonellin zusammen mit Vitamin B6 verwendet wird.

## Claims (Claims for the following Contracting State(s): AT, BE, CH, ES, FR, GB, GR, IT, LI, LU, NL, SE)

1. Use of trigonellin for the production of an encapsulated agent which is to be administered perorally for reviving, and for stimulating and enhancing hair growth in living creatures.

2. Use according to claim 1, wherein trigonellin is used together with vitamin B6.

3. Use according to claim 1 or 2, wherein trigonellin is used together with riboflavin and/or nicotinamide and/or calciumpantothenate and/or folic acid.

4. Use of trigonellin according to one of claims 1 to 3, wherein the trigonellin is obtained from fenugreek seed (trigonella phoenum graecum).

## Claims (Claims for the following Contracting State(s): DE)

1. Use of trigonellin together with riboflavin and/or nicotinamide and/or calciumpantothenate and/or folic acid for the production of an encapsulated agent which is to be administered perorally for reviving, and for stimulating and enhancing hair growth in living creatures.

2. Use of trigonellin according to claim 1, wherein the trigonellin is obtained from fenugreek seed (trigonella phoenum graecum).

3. Use of trigonellin according to claim 1 or claim 2, wherein trigonellin is used together with vitamin B6.

## Revendications (Revendications pour l'(les) Etat(s) contractant(s) suivant(s): AT, BE, CH, ES, FR, GB, GR, IT, LI, LU, NL, SE)

1. Utilisation de la trigonelline pour fabriquer un agent encapsulé destiné à être pris par voie orale, pour ranimer, stimuler et renforcer la pousse des cheveux chez le vivant.

2. Utilisation de la trigonelline selon la revendication 1, la trigonelline étant utilisée conjointement avec la vitamine B6.

3. Utilisation selon la revendication 1 ou 2, la trigonelline étant utilisée conjointement avec la riboflavine et/ou la nicotinamide et/ou le pantothénate de calcium et/ou de l'acide folique.

4. Utilisation de la trigonelline selon l'une des revendications 1 à 3, la trigonelline étant obtenue à partir de la graine de fenugrec (Trigonella phoenum graecum).

## Revendications (Revendications pour l'(les) Etat(s) contractant(s) suivant(s): DE)

1. Utilisation de la trigonelline conjointement avec la riboflavine et/ou la nicotinamide et/ou le pantothénate de calcium et/ou de l'acide folique pour fabriquer un agent encapsulé destiné à ètre pris par voie orale, pour ranimer, stimuler et renforcer la pousse de cheveux chez le vivant.

2. Utilisation de la trigonelline selon la revendication 1, la trigonelline étant obtenue à partir de la graine de fenugrec (Trigonella phoenum graecum).

3. Utilisation de la trigonelline selon la revendication 1 ou 2, la trigonelline étant utilisée conjointement avec la vitamine B6.
